# EUROPEAN PATENT APPLICATION

(11) **EP 1 946 724 A1**
(43) Date of publication of application: **23.07.2008**
(21) Application number: 06844001.5
(22) Date of filing: 08.12.2006
(51) Int. Cl.: A61F 2/24

(54) **HEART VALVE PROSTHESIS**

(30) Priority: 23.12.2005 RU 2005140363
(71) Applicant: Melnikov, Alexandr Petrovich, Moscow 109649 (RU); Melnikov, Denis Alexandrovich, Moscow 109469 (RU)
(72) Inventor: Melnikov, Alexandr Petrovich, Moscow 109649 (RU); Melnikov, Denis Alexandrovich, Moscow 109469 (RU)
(74) Representative: Engel, Christoph Klaus
(86) International application number: PCT/RU2006/000658
(87) International publication number: WO 2007/075121

(57) **Abstract**

This invention covers the field of medicine and is used in cardiosurgery in operations on replacement of affected natural heart valves. The prosthesis (1) includes a ring-shaped valve body (2), leaflets (3) with the possibility of turning and forming of a main zone of the flow area at the centre of the opening of the valve body (2) installed in the valve body (2) and the turning stops (18) of leaflets (3). The turning stops (18) of leaflets (3) represent two pairs of prominences (4, 5) located on the end surface (6) of the valve body (2) directed to the downstream flow of blood. The first pair of prominences (4) has the internal surface (7), at least a part of which is made flat and has a possibility to interact with flat sections (14) of leaflets (3) for restriction of the return flow of blood in the closed position. The prominences (4) on the opposite lateral sides (16) have guiding surfaces (8) interacting with bearing surfaces (9) of the lateral surface (11) of leaflets (3) by their turning. The generating lines of the guiding surface are inclined angularly to their internal surface and have a shape corresponding to the turning trajectory of the bearing surfaces of the leaflets (3) determining the axes of their turning by opening and closing of the prosthesis (1). Each prominence (5) of the second pair is located opposite a part of the lateral surface (11) of the corresponding leaflet (3) the most distant from the central surface (10) and is able to interact with its internal surface (15) with the mentioned part of the lateral surface (11) of the leaflet (3) during opening of the heart valve prosthesis. The turning limit stops (18) of the leaflets (3) are equipped with at least two rests (12) interacting with the external surface (13) of the leaflet (3) directed to the downstream flow of blood in its open position determining the turning angle of the leaflets. A design version of a tri-leaflet valve and execution variant of turning stops are disclosed.

## Description

### Technical field

This invention covers the field of medicine, in particular, cardiovascular surgery and is designed for replacement of affected natural heart valves during cardiosurgical operations.

### Prior knowledge

There are many designs of heart valves prostheses used for replacement of natural valves: ball, frameless, single leaflet, bi-leaflet, tri-leaflet prostheses. Widely used implanted valve body prostheses are very reliable and ensure satisfactory hemodynamics, appropriate improvements reduce acoustic noises, anxiety of patients, increase the time of continuous operation. Standard prostheses include a ring-shaped valve body providing a channel for stream of blood. One ore more leaflets is installed in the valve body with a possible repeat between the open position for passage of a direct stream of blood and the closed position for closure of a channel and restriction of the reverse stream of blood. In addition, the heart valve prosthesis contains a coupling arrangement for connection of a valve body with surrounding heart tissues. Basically, this arrangement is made in the form of a cuff which is sewed to the heart tissues and envelopes the lateral surface of the prosthesis' valve body.

Research and engineering activity is permanently carried out for creation of new designs of valve prostheses and modernization of existing ones by means of improvement of their features.

Efforts in the increase of efficiency of heart valves' prostheses are continued. The efficiency of the heart valve prosthesis is determined by the area of the flow section when the leaflets are in the open position. The valve body of the prosthesis sewed in with the help of a cuff into place of a removed natural valve has a certain thickness reducing the flow area. At that, the leaflets create the flow resistance and partially close a passage opening, especially by their central positioning. So, the effective area of the valve flow area is reduced, what is one of the main critical factors of the heart valve prostheses. This is particularly dangerous in valves with small diameters.

There are known from the prior art artificial heart valves which eliminate this disadvantage to a certain extent - these are the valves with an increased flow area and central positioning of leaflets by opening of the valve (see e.g. US patents N°N° 6007577, 6176877, 6719790). The increase of the flow area in these prostheses is achieved at the expense of the valve body having a flow area one size more (approx. 2 mm) as compared with a common valve and a cuff of a special design ensures the installation of such valve into a position over the excised natural valve. So, the thickness of the prosthesis valve body does not have any influence onto the change of the flow area of the valve and the flow area remains equal to the flow area of the natural valve it earlier has had.

However, a problem of hemodynamics distortion stays unsolved for such artificial valves. Indeed, the leaflets in open position of the valve stay in the central area of the velocity profile distorting it and reducing the blood velocity through the valve.

A series of designs is known (see US patent 4274437, RF patent 2066984) where the main flow area is executed in the centre. It is obvious by such design that the central part of the stream through the valve remains weakly disturbed and distortions caused by the leaflets are minimal. However, these valves have no practical application in the surgery because of low reliability and high probability of falling out of the leaflets. As it turned out, designing of such limit stops of the leaflets which could reliably hold the leaflets during the whole life of the valve and facilitate continuous operation of the valve represents the main difficulties.

So, a need for new design of the heart valve prosthesis combining the reliability and undistorted hemodynamics of the blood stream is obvious.

The closest analog for this invention is an artificial heart valve (RF 2066984) containing a ring-shaped valve body and two leaflets placed in it with a possibility of turning between top and bottom limit stops in relation to the blood stream and with a possibility of forming of a main zone of the flow area in the centre limited by round sections of inner walls of the valve body and bottom edges of the leaflets in relation to the blood stream, the bottom limit stops of each leaflet represent a pair of half-axles installed near the periphery of the valve area and the top limit stop of each leaflet is executed as a rest, the end of which is located in the groove of the rectangular section made on the peripheral edge of each leaflet directed to the rest.

### Disclosure of invention

This invention is directed to solution of a problem of creation of an artificial heart valve eliminating disadvantages of known analogs, namely having its main zone of the flow are at the centre and also having such a design which could ensure reliability and long continuous life of the valve.

The technical result obtained by use of this invention consists in reliability increase of holding the leaflets during the movement.

The technical result is obtained thanks to that that in the heart valve prosthesis containing a ring-shaped valve body, leaflets placed in it with a possibility of turning and forming of a main zone of the flow area in the centre installed inside the valve body and limit stops, the limit stops of leaflets turning represent two pairs of prominences located on the end surface of the valve body directed to the blood downstream, the first pair of prominences has an internal surface, at least a part of which is made flat and which is able to interact with flat sections of leaflets for restriction of the return flow of blood in the closed position, prominences on opposite sides have guiding surfaces interacting with corresponding bearing surfaces of the lateral surface of leaflets by their turning generating lines of the guiding surface of the prominences are inclined angularly to their internal surface and have a shape corresponding to the turning trajectory of the bearing surfaces of the leaflets determining the axes of their turning by opening and closing of the prosthesis, each prominence of the second pair is located opposite a part of the lateral surface of the corresponding leaflet the most distant from the central surface and is able to interact with its internal surface with the mentioned part of the leaflet's lateral surface during opening of the heart valve prosthesis, the limit stops for turning of the leaflets are equipped with at least two rests interacting with the external surface of the leaflet directed to the downstream flow of blood in its open position determining the turning angle of the leaflets.

The technical result increases thanks to that that the mentioned flat sections of the leaflets are located on the lateral surface of each leaflet from two diametrically opposite sides and have a possibility of interaction with the corresponding flat surface of the turning stop of the leaflet in closed position, at that, each flat section of the leaflet ends with a bearing surface, generating lines of which are inclined towards the flat surfaces, at that, the bearing surfaces of the leaflets have a possibility of interaction with the corresponding guiding surfaces of the turning stops of the leaflets during opening and closing of the heart valve prosthesis.

In one version of execution of the turning stops of the leaflets, the first pair of prominences is equipped with four rests which are pairwise-located on the internal surface of the prominences from a side directed to the downstream flow of blood.

In other version of execution, the first pair of prominences is equipped with four rests which are pairwise-located on the opposite lateral sides of the first pair of prominences from a side directed to the downstream flow of blood.

The third variant is characterized by that that there are two rests in turning limit stops, each of them is located on the internal surface of the second pair from side directed to the downstream flow of blood.

The internal surface of an opening of a ring-shaped valve body is made as a lateral surface of the circular cylinder and limited with two flat ends directed to the upstream and downstream flows of blood.

Two hollows are made on the end directed to the downstream flow of blood, the surfaces of each hollow serve for interaction with a part of the lateral surface of the corresponding leaflet the most distant from the central surface by opening and closing of the prosthesis and forming of a limited return stream of blood in its closed position.

A circular groove for fastening of an inserted cuff is made on the external side of the valve body.

The external surface of each leaflet directed to the downstream flow of blood is made at least partially concave and the external surface of each leaflet directed to the upstream flow of blood is made at least partially convex, at that, generating lines of the mentioned parts of surfaces are inclined angularly to the central surface of the leaflet.

The external surface of each leaflet directed to the downstream flow of blood is made at least partially concave and the external surface of each leaflet directed to the upstream flow of blood is made at least partially convex, at that, axes of symmetry of the mentioned parts of surfaces are inclined angularly to the central axis of the ring-shaped valve body.

The convex and concave parts of the external surfaces of the leaflets represent lateral surfaces of the circular cylinder inclined relative the central axis of the heart valve prosthesis.

All valve parts are made of pyrocarbon.

In addition, the technical result is obtained thanks to that that in the heart valve prosthesis including a ring-shaped valve body, three leaflets with a possibility of turning and forming of a main zone of the flow area in the centre of the valve body opening installed inside the valve body and the limit stops, the limit stops of leaflets turning represent two pairs of three prominences located on the end surface of the valve body directed to the blood downstream flow, the first three prominences have bearing surfaces on their opposite lateral sides which interact with corresponding guiding surfaces of the lateral surface of leaflets by opening and closing of the prosthesis, each prominence of the second three prominences is located opposite a part of the lateral surface of the corresponding leaflet the most distant from the central surface and is able to interact with its internal surface with the mentioned part of the leaflet's lateral surface during opening of the heart valve prosthesis, the limit stops for turning of the leaflets are equipped with at least two rests interacting with the external surface of the leaflet directed to the downstream flow of blood in its open position determining the turning angle of the leaflets.

### Brief description of figures

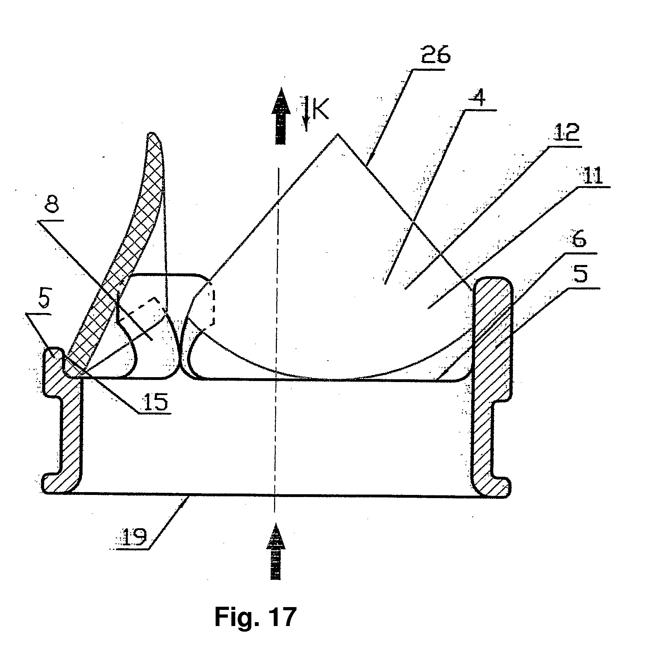

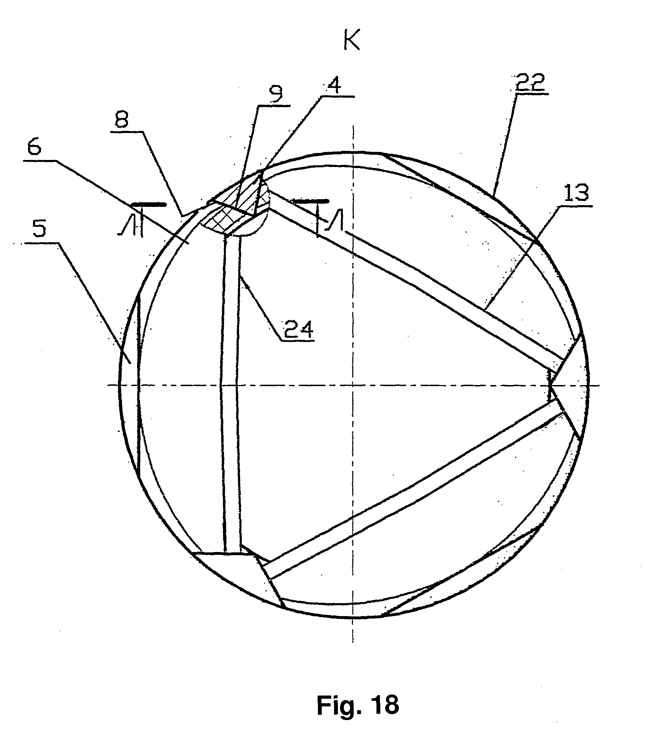

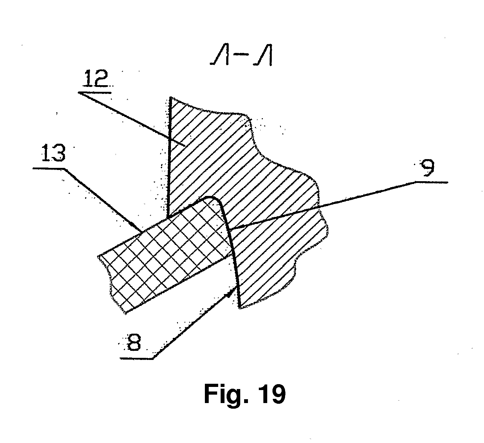

The character of the invention will be more clear from a description given below with references to drawing items where a valve having repeat stops of the leaflets of the version 1 is shown in Fig. 1 - 5: front view of the valve with closed leaflets, the same with open leaflets, side view, view from above, view in section on B-B showing the interaction of the rest and the leaflet in its open position accordingly, a valve with turning stops of the version 2 is shown in Fig. 6 -10: front view of the valve with closed leaflets, the same with open leaflets, side view, view from above, view in section on 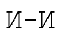 showing the interaction of the rest and the leaflet accordingly, a valve with limit stops of the version 3 is shown in Fig. 11 - 15: front view of the valve with closed leaflets, the same with open leaflets, side view, view from above, cut E showing the interaction of hollows of the rest and the leaflet accordingly, a structure of a tri-leaflet valve is shown in Fig. 16 - 19, in Fig. 16 - front view of the valve with closed leaflets, in Fig. 17 - front view of the valve with open leaflets, in Fig. 18 - view from above, in Fig. 19 - section on JI-JI on the view from above showing the interaction of the external surface of the leaflet with the rest accordingly.

### Embodiment of invention

The heart valve prosthesis 1 contains a ring-shaped valve body 2 made of pyrocarbon and having two or three leaflets 3 also made of pyrocarbon and with the possibility of turning and forming of a main zone of the flow area at the centre installed in the valve body 2. The turning stops 18 of leaflets 3 are made jointly with the valve body and represent two pairs of prominences - the first pair of prominences 4 and the second pair of prominences 5 located on the end surface 6 of the valve body 2 , directed to the downstream flow of blood (indicated with down arrow in drawings). In the version of a tri-leaflet valve, the stops represent two pairs of three prominences accordingly. A design version of the bi-leaflet valve will be described further for the sake of simplicity. The first pair of prominences 4 has the internal surface 7, a part of which is made flat and interacts with flat sections of leaflets 3 for restriction of the return flow of blood in the closed position. The prominences 4, 5 on the opposite lateral sides have guiding surfaces 8 interacting with bearing surfaces 9 of the lateral surface of leaflets 3 by their turning. The generating lines of the guiding surface of the prominences 4 and 5 are inclined angularly to their internal surface and have a shape corresponding to the turning trajectory of the bearing surfaces 9 of the leaflets 3 determining the axes of their turning by opening and closing of the prosthesis 1. Each prominence 5 of the second pair is located opposite a part of the lateral surface 11 of the corresponding leaflet 3 the most distant from the central surface 10 and is able to interact with its internal surface with the mentioned part of the lateral surface 11 of the leaflet 3 during opening of the heart valve prosthesis 1. The turning limit stops 18 of the leaflets 3 are equipped with rests 12 interacting with the external surface 13 of the leaflet 3 directed to the downstream flow of blood in its open position determining the turning angle of the leaflet 3.

Flat sections 14 of the leaflets 3 are located on the lateral surface of each leaflet 3 from two diametrically opposite sides and these sections interact with the corresponding flat surface 15 of the turning stop of the leaflet 3 in closed position. Each flat section 14 of the leaflet 3 ends with a bearing surface 9, generating lines of which are inclined towards the flat surfaces 15. The bearing surfaces 9 of the leaflets interact with the corresponding guiding surfaces 8 of the turning stops 18 of the leaflets 3 during opening and closing of the heart valve prosthesis 1.

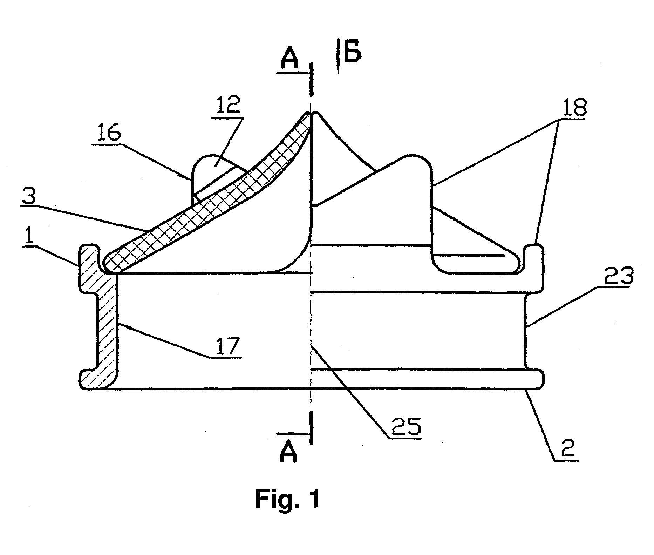

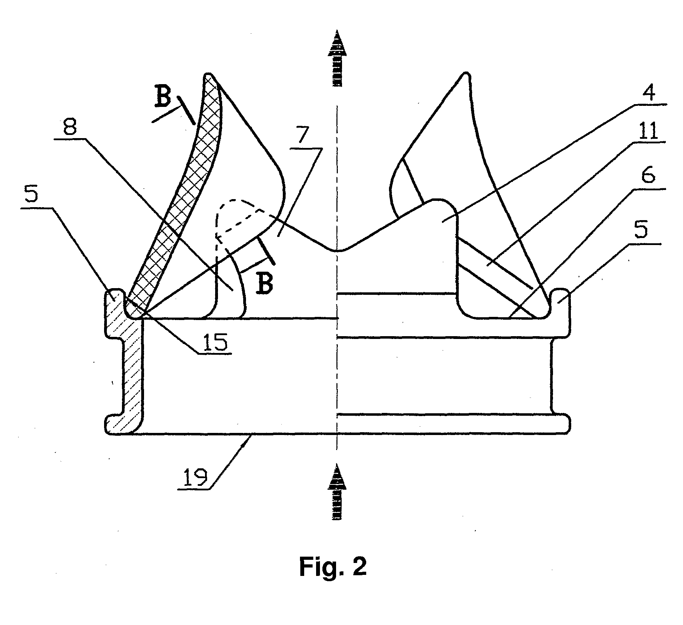

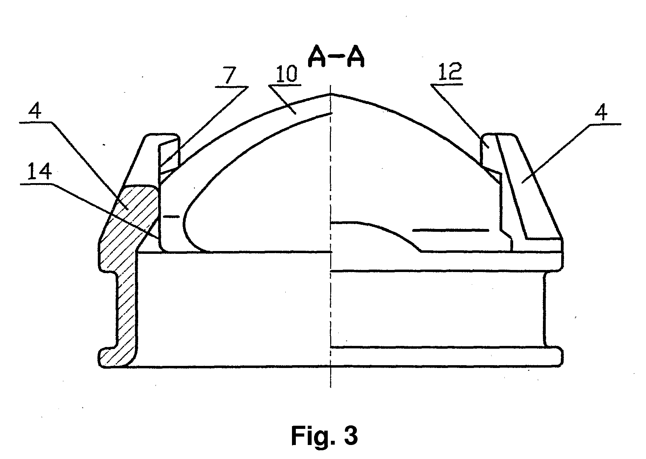

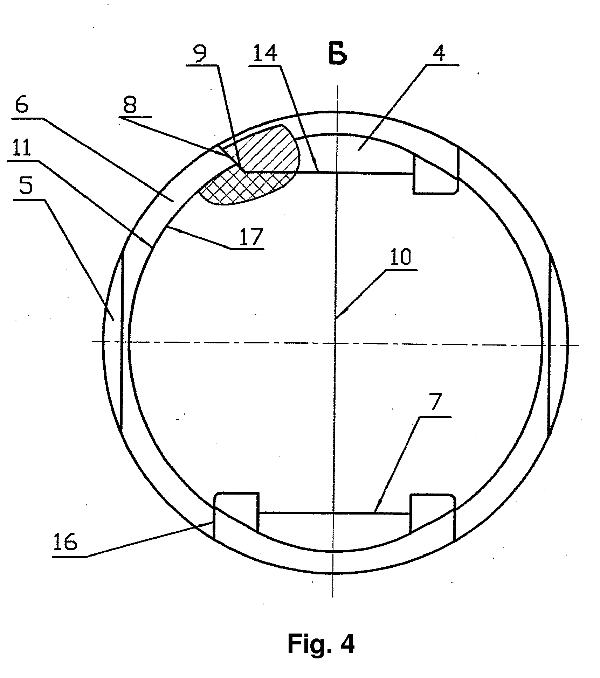

In one version of execution (Fig. 1 - 5) of the turning stops 18, the first pair of prominences 4 is equipped with four rests 12 which are pairwise-located on the internal surface 7 of the prominences 4 from a side directed to the downstream flow of blood.

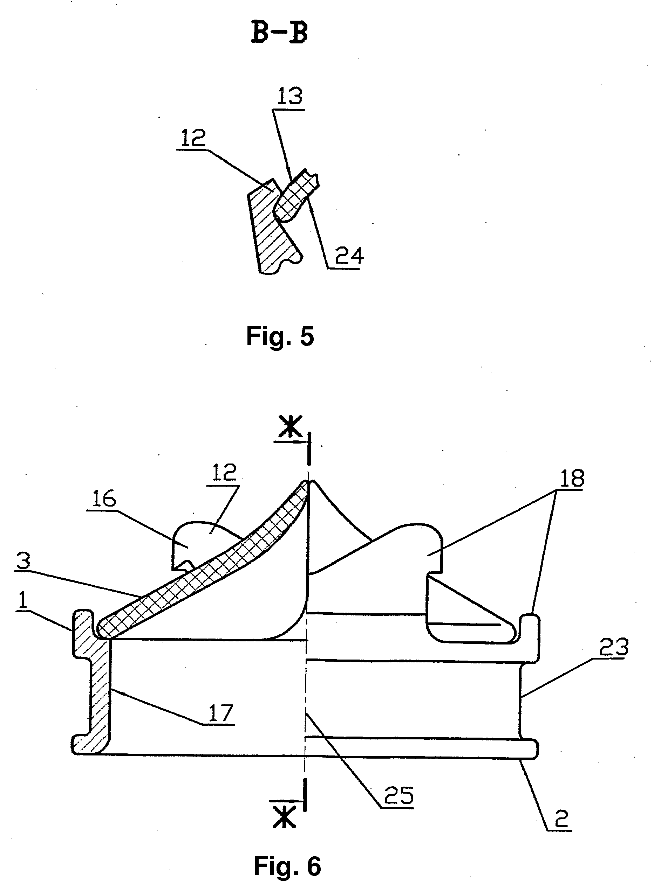

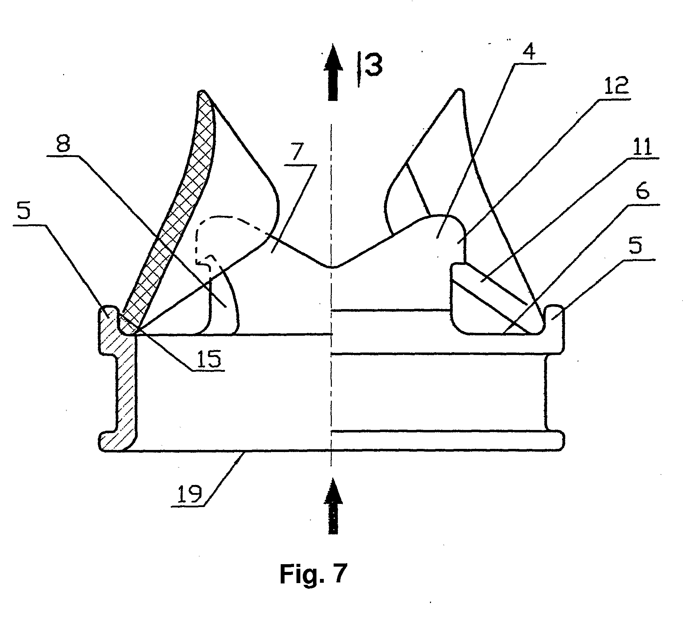

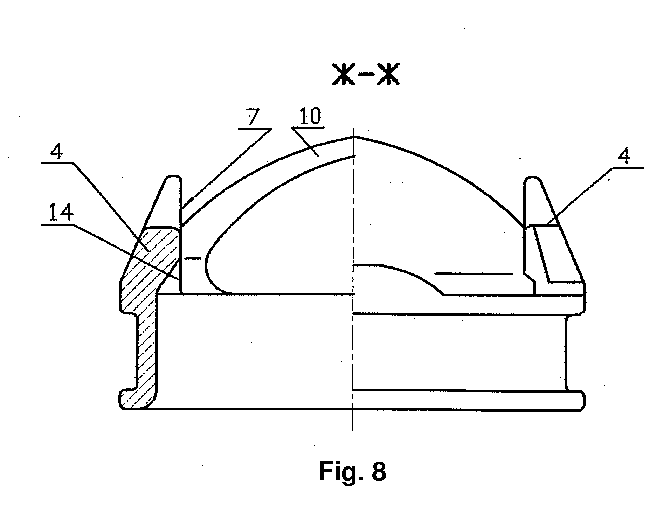

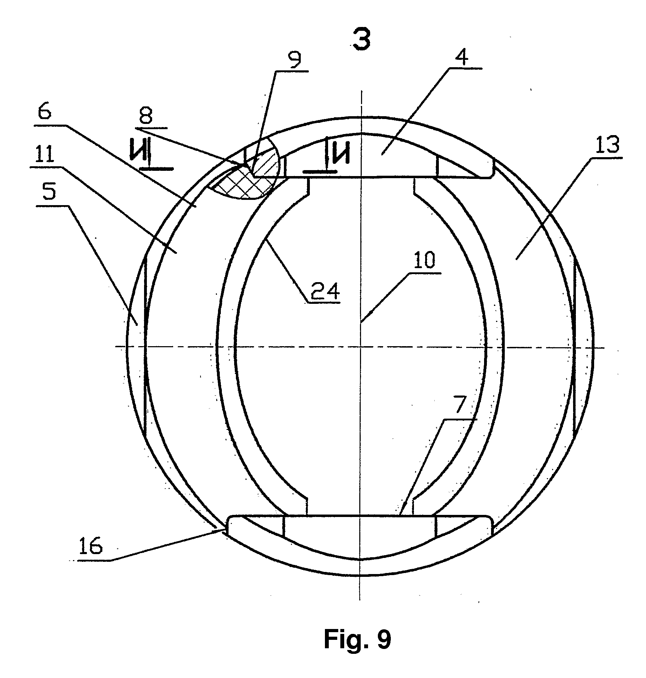

In other version of execution (Fig. 5 - 10), the first pair of prominences 4 is equipped with four rests 12 which are pairwise-located on the opposite lateral sides 16 of the first pair of prominences 4 from a side directed to the downstream flow of blood.

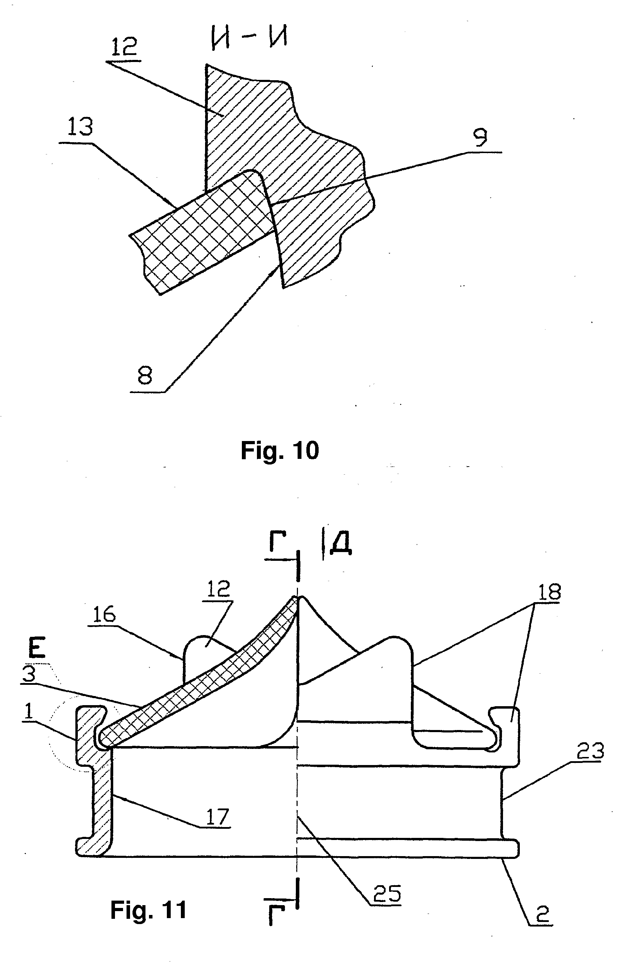

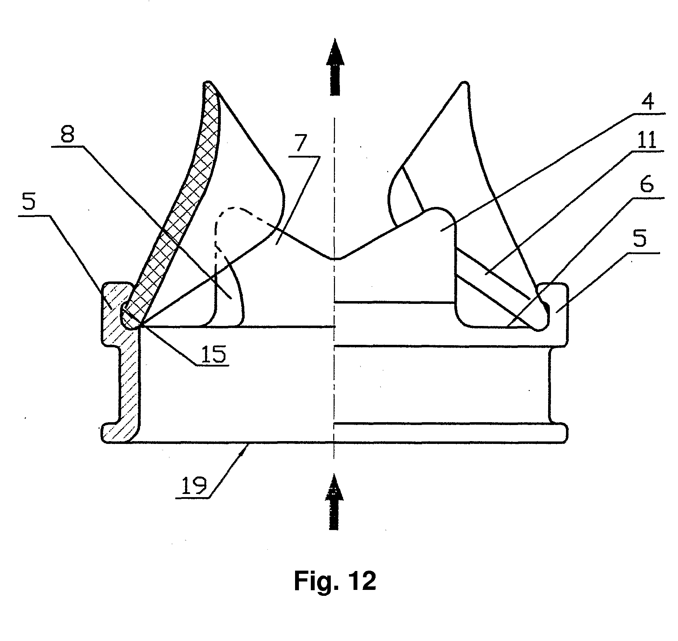

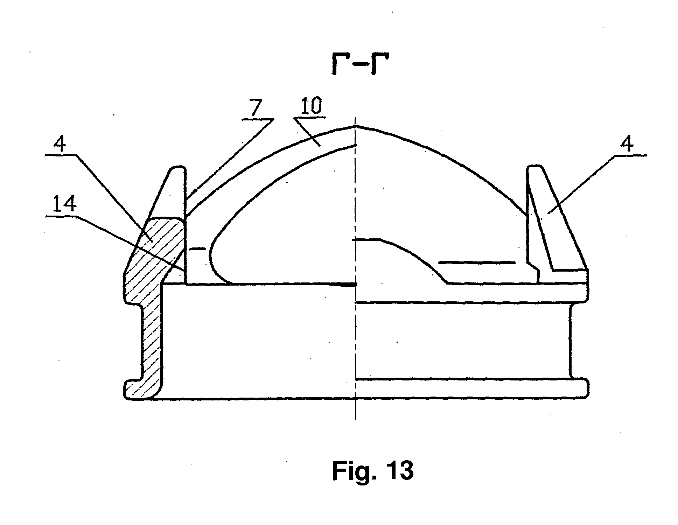

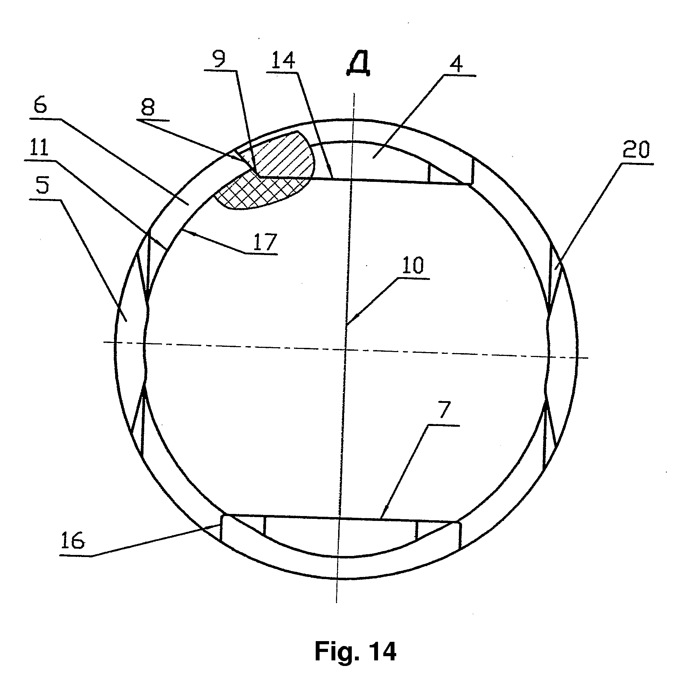

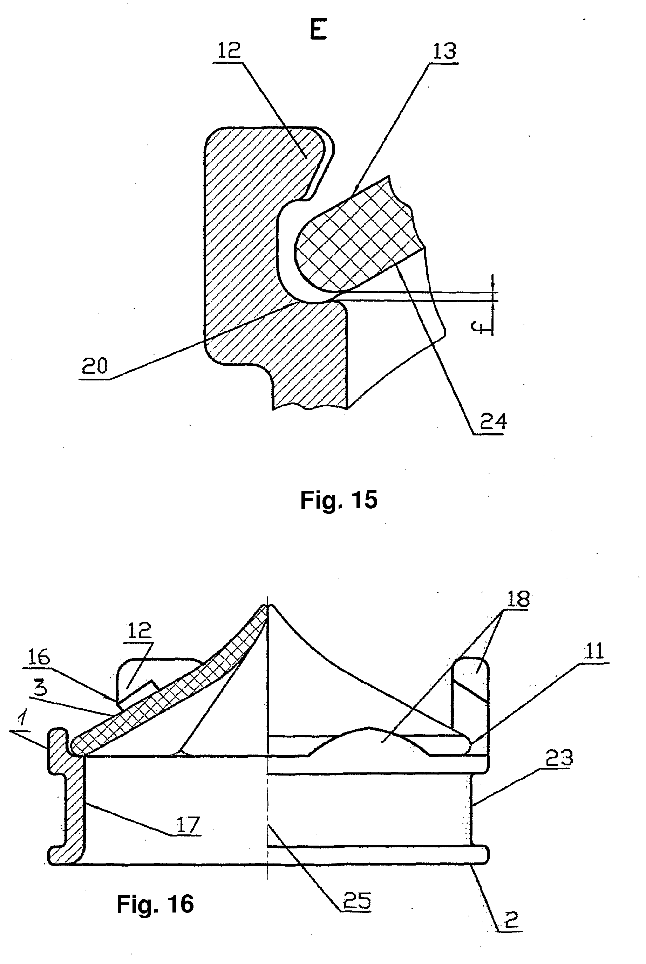

The third design variant (Fig. 11 - 15) has two rests 12 of turning limit stops 18, each of the rest 12 is located on the internal surface of the second pair of prominences 5 from side directed to the downstream flow of blood.

The surface of the opening 17 of a ring-shaped valve body 2 is made as a lateral surface of the circular cylinder and limited with two flat ends 19 and 6 directed to the upstream and downstream flows of blood (indicated with up and down arrows accordingly).

Two hollows 20 are made on the end 6 directed to the downstream flow of blood. The surfaces of each hollow 20 serve for interaction with a part 10 of the lateral surface 11 of the corresponding leaflet 3 the most distant from the central surface 10 by opening and closing of the prosthesis 1 and forming of a limited return stream of blood in its closed position.

A circular groove 23 for fastening of an inserted cuff (not shown) is made on the external surface 22 of the valve body 2.

The external surface 13 of each leaflet 3 directed to the downstream flow of blood is made at least partially concave. The external surface 24 of each leaflet directed to the upstream flow of blood is made at least partially convex. The generating lines of the mentioned parts of surfaces are inclined angularly to the central surface 21 of the leaflet 3.

The axes of symmetry of the mentioned parts of surfaces 13 and 24 are inclined angularly to the central axis 25 of the ring-shaped valve body 2.

The convex and concave parts of the external surfaces 13 and 24 of the leaflets 3 represent lateral surfaces of the circular cylinder inclined relative the central axis 25 of the heart valve prosthesis 1.

The heart valve prosthesis operates as follows. By opening of the valve prosthesis 1 the action of the direct stream of blood causes turning of leaflets 3 around the imaginary axis determined by the rests 12. At that, the rests 12 interact with the external surfaces of the leaflets 3. This turn occurs until a balanced state position. At that, the blood flows into the upright direction, the central part of the opening 17 of the valve body 2 passes the stream freely and without distortion. The leaflets are located practically vertically and do not create any resistance. The rests 12 of the turning stops 18 of the leaflets 3 interacts with the external surface 13 of the leaflets and reliably hold them in the open position.

The convexo-concave execution of external surfaces of the leaflets provides even more opening of the valve. By stopping of the direct flow the leaflets 3 begin to turn and return in their original position until the moment of contact each other with the central surfaces 21.

### Industrial applicability

The artificial heart valve can be industrially manufactured of materials established and widely applied in medicine and on the well-known equipment.
The prosthesis applied for patent has the following advantages by application in the cardiosurgery for replacement of function of affected natural valves:
1. It ensures high efficiency of the flow area of the valve by showing practically no resistance to the flow in the open position of the leaflets.
2. It excludes the forming of congestive areas thanks to washing of location areas of the flow limit stops and rests by the direct and return stream of blood.
3. It significantly reduces the factor of thrombi formation.
4. It excludes the instability of the valve by opening thanks to the convexo-concave execution of external surfaces of the leaflets.
5. It excludes the falling out of the leaflets thanks to the execution of the turning stops with rests.
6. It excludes the turning of the leaflets under the influence of the return stream of blood thanks to resting of the external surface of the leaflet onto the limit stops and interaction of the central lateral surfaces and, consequently, it excludes regurgitation of the valve.
7. It has high reliability and life duration, good compatibility with natural tissues.

## Claims

1. Heart valve prosthesis including a ring-shaped valve body, leaflets with a possibility of turning and forming of a main zone of the flow area in the centre of the valve body opening installed inside the valve body and turning limit stops of leaflets **characterized in that**:
- the turning limit stops represent two pairs of prominences located on the end surface of the valve body directed to the blood downstream,
- the first pair of prominences has an internal surface, at least a part of which is made flat and which is able to interact with flat sections of leaflets for restriction of the return flow of blood in the closed position, prominences on opposite sides have guiding surfaces interacting with corresponding bearing surfaces of the lateral surface of leaflets by their opening and closing,
- the guiding surfaces are made by turning of their generating line relative the turning axis of the leaflets,
- each prominence of the second pair is located opposite a part of the lateral surface of the corresponding leaflet the most distant from the central surface and is able to interact with its internal surface with the mentioned part of the leaflet's lateral surface during opening of the heart valve prosthesis,
- the limit stops for turning of the leaflets are equipped with at least two rests interacting with the external surface of the leaflet directed to the downstream flow of blood in its open position determining the turning angle of the leaflets.

2. Prosthesis according to calim 1 **characterized in that** the mentioned flat sections of the leaflets are located on the lateral surface of each leaflet from two diametrically opposite sides and have a possibility of interaction with the corresponding flat surface of the turning stop of the leaflet in the closed position, at that, each flat section of the leaflet ends with a bearing surface, generating lines of which are inclined towards the flat surfaces, and that the bearing surfaces of the leaflets have a possibility of interaction with the corresponding guiding surfaces of the turning stops of the leaflets during opening and closing of the heart valve prosthesis.

3. Prosthesis according to claim 1 or 2 **characterized in that** the generating lines of the guiding surface of the first pair of prominences are inclined angularly to their internal surface, and that the angle of inclination corresponds to the angle of inclination of the generating line of the leaflets' bearing surface.

4. Prosthesis according to claim 1 **characterized in that** the first pair of prominences is equipped with four prominences which are pairwise-located on the internal surface of the prominences from a side directed to the downstream flow of blood.

5. Prosthesis according to claim 1 **characterized in that** the first pair of prominences is equipped with four rests which are pairwise-located on the opposite lateral sides of the first pair of prominences from a side directed to the downstream flow of blood.

6. Prosthesis according to claim 1 **characterized in that** two rests in turning limit stops are located on the internal surface of the second pair from side directed to the downstream flow of blood.

7. Prosthesis according to claim 1 **characterized in that** the internal surface of an opening of a ring-shaped valve body is made as a lateral surface of the circular cylinder and limited with two flat ends directed to the upstream and downstream flows of blood.

8. Prosthesis according to claim 1 or 6 **characterized in that** two hollows are made on the end directed to the downstream flow of blood, the surfaces of each hollow serve for interaction with a part of the lateral surface of the corresponding leaflet the most distant from the central surface by opening and closing of the prosthesis and forming of a limited return stream of blood in its closed position.

9. Prosthesis according to claim 1 **characterized in that** a circular groove for fastening of an inserted cuff is made on the external side of the valve body.

10. Prosthesis according to claim 1 **characterized in that** the external surface of each leaflet directed to the downstream flow of blood is made at least partially concave and the external surface of each leaflet directed to the upstream flow of blood is made at least partially convex, and that generating lines of the mentioned parts of surfaces are inclined angularly to the central surface of the leaflet.

11. Prosthesis according to claim 1 **characterized in that** the external surface of each leaflet directed to the downstream flow of blood is made at least partially concave and the external surface of each leaflet directed to the upstream flow of blood is made at least partially convex, and that axes of symmetry of the mentioned parts of surfaces are inclined angularly to the central axis of the ring-shaped valve body.

12. Prosthesis according to claim 9 or 10 **characterized in that** the convex and concave parts of the external surfaces of the leaflets represent lateral surfaces of the circular cylinder inclined relative the central axis of the heart valve prosthesis.

13. Prosthesis according to any of claims 1 -7, 9- 11 **characterized in that** it is made of pyrocarbon.

14. Heart valve prosthesis including a ring-shaped valve body, three leaflets with a possibility of turning and forming of a main zone of the flow area in the centre of the valve body opening installed inside the valve body and the limit stops, the limit stops of leaflets turning **characterized in that**:
- the limit stops represent two pairs of three prominences located on the end surface of the valve body directed to the downstream flow of blood,
- the first three prominences have bearing surfaces on their opposite lateral sides which interact with corresponding guiding surfaces of the lateral surface of leaflets by opening and closing of the prosthesis,
- each prominence of the second three prominences is located opposite a part of the lateral surface of the corresponding leaflet the most distant from the central surface and is able to interact with its internal surface with the mentioned part of the leaflet's lateral surface during opening of the heart valve prosthesis,
- the limit stops for turning of the leaflets are equipped with at least two rests interacting with the external surface of the leaflet directed to the downstream flow of blood in its open position determining the turning angle of the leaflets.

15. Prosthesis according to claim 14 **characterized in that** the generating lines of the guiding surface of the first pair of prominences are inclined angularly to their internal surface, and that the angle of inclination corresponds to the angle of inclination of the generating line of the leaflets' bearing surface.

16. Prosthesis according to claim 14 **characterized in that** the first three prominences are equipped with four rests which are pairwise-located on the internal surface of the prominences from a side directed to the downstream flow of blood.

17. Prosthesis according to claim 14 **characterized in that** the first three prominences are equipped with four rests, and that the rests of the first three prominences are pairwise-located on the opposite lateral sides of the first three prominences from a side directed to the downstream flow of blood.

18. Prosthesis according to claim 14 **characterized in that** two rests of the limit stops are located on the internal surface of the second three prominences from the side directed to the downstream flow of blood.

19. Prosthesis according to claim 14 **characterized in that** the internal surface of an opening of a ring-shaped valve body is made as a lateral surface of the circular cylinder and limited with two flat ends directed to the upstream and downstream flows of blood.

20. Prosthesis according to claim 14 or 19 **characterized in that** two hollows are made on the end directed to the downstream flow of blood, the surfaces of each hollow serve for interaction with a part of the lateral surface of the corresponding leaflet the most distant from the central surface by opening and closing of the prosthesis and forming of a limited return stream of blood in its closed position.

21. Prosthesis according to claim 14 **characterized in that** a circular groove for fastening of an inserted cuff is made on the external side of the valve body.

22. Prosthesis according to claim 14 **characterized in that** the external surface of each leaflet directed to the downstream flow of blood is made at least partially concave and the external surface of each leaflet directed to the upstream flow of blood is made at least partially convex, and that generating lines of the mentioned parts of surfaces are inclined angularly to the central surface of the leaflet.

23. Prosthesis according to claim 14 **characterized in that** the external surface of each leaflet directed to the downstream flow of blood is made at least partially concave and the external surface of each leaflet directed to the upstream flow of blood is made at least partially convex, and that axes of symmetry of the mentioned parts of surfaces are inclined angularly to the central axis of the ring-shaped valve body.

24. Prosthesis according to claim 21 or 23 **characterized in that** the convex and concave parts of the external surfaces of the leaflets represent lateral surfaces of the circular cylinder inclined relative the central axis of the heart valve prosthesis.

25. Prosthesis according to any of claims 14 -19, 20- 23 **characterized in that** it is made of pyrocarbon.
